Europäisches Patentamt

European Patent Office

Office européen des brevets

(19)

(11) Veröffentlichungsnummer : **0 095 630**
**B1**

(12) **EUROPÄISCHE PATENTSCHRIFT**

(45) Veröffentlichungstag der Patentschrift :
05.03.86

(51) Int. Cl.⁴ : **C 12 M   1/30**

(21) Anmeldenummer : **83104745.1**

(22) Anmeldetag : **13.05.83**

(54) **Sammel- und Transportvorrichtung für Mikroorganismen.**

(30) Priorität : **28.05.82 DE 3220139**

(43) Veröffentlichungstag der Anmeldung :
**07.12.83 Patentblatt 83/49**

(45) Bekanntmachung des Hinweises auf die Patenterteilung : **05.03.86 Patentblatt 86/10**

(84) Benannte Vertragsstaaten :
**BE CH DE FR GB IT LI NL SE**

(56) Entgegenhaltungen :
**AU-A-   517 787**
**FR-A- 2 173 259**
**FR-A- 2 336 483**
**US-A- 4 211 323**
**Die Akte enthält technische Angaben, die nach dem
Eingang der Anmeldung eingereicht wurden und die
nicht in dieser Patentschrift enthalten sind.**

(73) Patentinhaber : **Merck Patent Gesellschaft mit beschränkter Haftung
Frankfurter Strasse 250
D-6100 Darmstadt (DE)**

(72) Erfinder : **Kappner, Manfred, Dr.
Georg-Büchner-Strasse 26
D-6107 Reinheim 3 (DE)**
Erfinder : **Gunkel, Werner
Wingertsweg 33
D-6101 Rossdorf 2 (DE)**
Erfinder : **Trinkaus, Winfried
Holunderweg 13
D-6110 Dieburg (DE)**
Erfinder : **Bauer, Roland
Adam-Schwinn-Strasse 13
D-6102 Pfungstadt (DE)**

# Beschreibung

Die Erfindung betrifft eine Sammel- und Transportvorrichtung für Mikroorganismen, die im wesentlichen aus einem Tiefziehteil besteht, das einen Abstrichtupfer und einen speziellen Kulturmediumbehälter enthält. Die Vorrichtung dient dazu, die mit dem Abstrichtupfer z. B. in Arztpraxen durch Abstriche gewonnenen Proben zur mikrobiologischen Untersuchung in ein ggf. örtlich getrenntes Untersuchungslabor befördern zu können.

Die mikrobiologische Zusammensetzung einer Untersuchungsprobe muß dabei sowohl qualitativ als auch quantitativ von der Entnahme bis zur Weiterverarbeitung im Untersuchungslabor möglichst konstant gehalten werden. Der Zeitraum zwischen Probenentnahme und Untersuchung in einem mikrobiologischen Labor sollte nach Möglichkeit 48 Stunden nicht überschreiten. Es müssen nicht nur empfindliche Mikroorganismen beim Transport vor dem Absterben geschützt, sondern auch weniger empfindliche Mikroorganismen an der Vermehrung gehindert werden.

Bei einer Vermehrungsmöglichkeit für Keime beim Transport könnten sich z. B. bei der Probenentnahme eingeschleppte Kontaminationskeime schneller vermehren als die nachzuweisenden Mikroorganismen. Dadurch wird es beim Eintreffen der Proben im Untersuchungslabor sehr schwierig, die eigentlichen Krankheitserreger von mikrobiologischen Kontaminationen abzugrenzen oder die Erreger überhaupt unter den überwuchernden Kontaminationen aufzuspüren. Das Kulturmedium der Transportvorrichtung sollte deshalb eine Art Ruhemilieu ohne wesentliche Nährstoff- und Energiequellen sein.

Zum Sammeln und Transport von Mikroorganismen sind z. B. Vorrichtungen bekannt, bei denen der Abstrichtupfer und das Kulturmedium hintereinander in einer mit einem wasserdichten Papierstreifen verschlossenen, muldenartig verformten Kunststoffolie untergebracht sind (US-A-3 835 834). Abstrichtupfer und Kulturmedium sind durch einen Steg voneinander getrennt. Durch Druck von außen auf den Kulturmediumbehälter öffnet sich der Trennsteg und das Kulturmedium gelangt zum Wattebausch des Abstrichtupfers. Bei dieser Vorrichtung ist die Ausbildung des Stegs zum Austritt des Kulturmediums aus dem Behälter problematisch. Die Haltbarkeit des Kulturmediums ist von der Gas- und Wasserdampfdichtigkeit des verwendeten Kunststoffs und des wasserdichten Papiers abhängig.

Aus dem US Patent 4 211 323 ist eine Vorrichtung bekannt, bei der ein mit einem flüssigen Kulturmedium gefüllten Kulturmediumbehälter durch Druck von außen geöffnet werden kann ; die Vorrichtung enthält eine abnehmbare Verschlußkappe.

Bei einer weiteren Vorrichtung zum Sammeln und Transportieren von Mikroorganismen, die von einem Kunststoffröhrchen ausgeht, wird zur Aufbewahrung des Kulturmediums eine Glasampulle verwendet (DE-B-26 36 462). Nach dem Abstrich und der Einführung des Abstrichtupfers in das Kunststoffrohr wird die Glasampulle durch Druck von außen auf das Kunststoffrohr zerbrochen. Das flüssige Kulturmedium wird von einem Wattestopfen aufgesaugt.

Der Wattebausch des Abstrichtupfers ist partiell von dem mit Kulturmedium getränkten Wattestopfen umschlossen. Die Aufbewahrung des Kulturmediums in der Glasampulle gewährleistet zwar gute Haltbarkeitszeiten des Kulturmediums, jedoch ist eine Verletzungsgefahr durch Glassplitter beim Zerbrechen der Glasampulle nicht sicher auszuschließen.

Bei den bekannten Systemen ist je nach deren Ausführung die Herstellung sehr aufwendig, die Haltbarkeit begrenzt oder die Handhabung unsicher.

Der Erfindung lag die Aufgabe zugrunde, eine Sammel- und Transportvorrichtung für Mikroorganismen zu schaffen, die die Nachteile der bekannten Vorrichtungen nicht aufweist, die einfach und preiswert herzustellen ist, eine hohe Qualitätskonstanz erreicht, eine lange Haltbarkeit des Kulturmediums garantiert und eine einfache und sichere Handhabung gewährleistet. Diese Aufgabe wurde vor allem durch die spezielle Gestaltung des Kulturmediumbehälters gelöst.

Gegenstand der Erfindung ist eine Sammel und Transportvorrichtung für Mikroorganismen, bestehend im wesentlichen aus einem einen Abstrichtupfer (2) und einen abgeschlossenen Kulturmediumbehälter (3) enthaltenden Tiefziehteil (1), das an der Oberseite teils mit einer aufgeschweißten Deckfolie (4) und mit einer abziehbaren und wieder dicht verschließbaren Klebefolie (5) verschlossen ist, die dadurch gekennzeichnet ist, daß das Kulturmedium (9) in einem separaten abgeschlossenen, im Tiefziehteil (1) gelagerten Behälter (3) untergebracht ist und Behälter (3), Tiefziehteil (1), Deckfolie (4) und Klebefolie (5) aus Folien geringer Gas- und Wasserdampfdurchlässigkeit bestehen.

Vorzugsweise besteht der Kulturmediumbehälter (3) aus einem Formteil aus Kunststoff mit äußerst geringer Gas- und Wasserdampfdurchlässigkeit wie Polypropylen, Polyethylen, Polymethylpentan usw. Auf den Kulturmediumbehälter (3) ist eine mit dem Abstrichtupfer leicht zu durchstoßende Folie (6) mit äußerst geringer Gas- und Wasserdampfdurchlässigkeit aufgesiegelt. Eine mit einem Lack beschichtete etwa 10-15 $\mu$m dicke Aluminiumfolie eignet sich vorzugsweise als leicht zu durchstoßende Folie (6) für den Kulturmediumbehälter.

Der Kulturmediumbehälter kann auch aus zwei Aluminiumverbundfolien mit einer Dicke von etwa 50-200 $\mu$m bestehen, in die ein- oder beidseitig muldenartige Vertiefungen geformt sind. Beide Folien sind entweder vollständig oder bis auf eine kleine Aussparung (11) rundherum miteinander versiegelt oder verschweißt. Die Aussparung, die als präformierte Austrittsstelle des Kulturmediums dient, wird mit einem gezahnten Prägewerkzeug flüssigkeitsdicht verschlossen.

Der eine abgeschlossene Einheit darstellende, dicht verschlossene Kulturmediumbehälter (3) gewährleistet eine lange Haltbarkeit des Kulturmediums ohne signifikante qualitative und quantitative Veränderungen, selbst bei längerer Lagerung bei Zimmertemperatur.

Die Tiefziehfolie (1), die aufgeschweißte Deckfolie (4) sowie die Klebefolie (5) sollen ebenfalls aus Materialien mit möglichst geringer Gas- und Wasserdampfdurchlässigkeit bestehen. Als Tiefziehfolien können etwa 200-500 μm dicke Folien aus Polyvinylchlorid, Polypropylen oder Polyethylen verwendet werden, als Deck- und Klebefolien etwa 50-200 μm dicke Folien aus den gleichen Materialien. Bei der Klebefolie wird die Kunststoffolie mit einem geeigneten Klebstoff, z. B. auf Acrylharzbasis, beschichtet. Alle im Sammel- und Transportsystem verwendeten Materialien müssen gegen eine γ-Bestrahlung mit 2,5 MRad beständig sein.

Sowohl der Kulturmediumbehälter als auch das Tiefziehteil und die Deck- und Klebefolie bestehen aus Materialien mit möglichst geringer Gas- und Wasserdampfdurchlässigkeit. Dadurch wird gewährleistet, daß das Kulturmedium im Kulturmediumbehälter selbst bei einer Lagerung bei Zimmertemperatur eine lange Haltbarkeit hat ; durch die geringe Gas- und Wasserdampfdurchlässigkeit des Tiefziehteils, der Deck- und Klebefolie wird eine gute Überlebensrate der abgestrichenen Mikroorganismen — auch bei einem Transport unter anaeroben Bedingungen — gewährleistet.

Als Tupfer zur Gewinnung von Abstrichmaterial für die mikrobiologische Untersuchung sind alle herkömmlichen Tupfer geeignet, die keine Hemmstoffe für Mikroorganismen enthalten und sich in wäßrigen Medien nicht auflösen. Vorzugsweise werden Tupfer aus Polyethylenterephthalat oder regenerierter Cellulose (Rayon) eingesetzt. Tupfer aus Baumwolle sind weniger gut geeignet, da sie häufig Fettsäurespuren enthalten, die toxisch für Mikroorganismen sind.

Durch eine richtige Abstimmung der Kulturmediummenge auf die Abmessungen des Abstrichtupfers wird eine sichere Benetzung des Wattebausches mit Kulturmedium gewährleistet. In einer bevorzugten Ausführungsform enthält die erfindungsgemäße Vorrichtung bei einer Gesamtlänge des Sammel- und Transportsystems von etwa 15 bis 30 cm einen Kulturmediumbehälter mit etwa 1 bis 10 ml Kulturmedium. Durch Beilage eines Reagenziensystems zur Erzeugung eines anaeroben Milieus in die Tiefziehpackung ist die erfindungsgemäße Vorrichtung auch für den anaeroben Transport von Mikroorganismen geeignet.

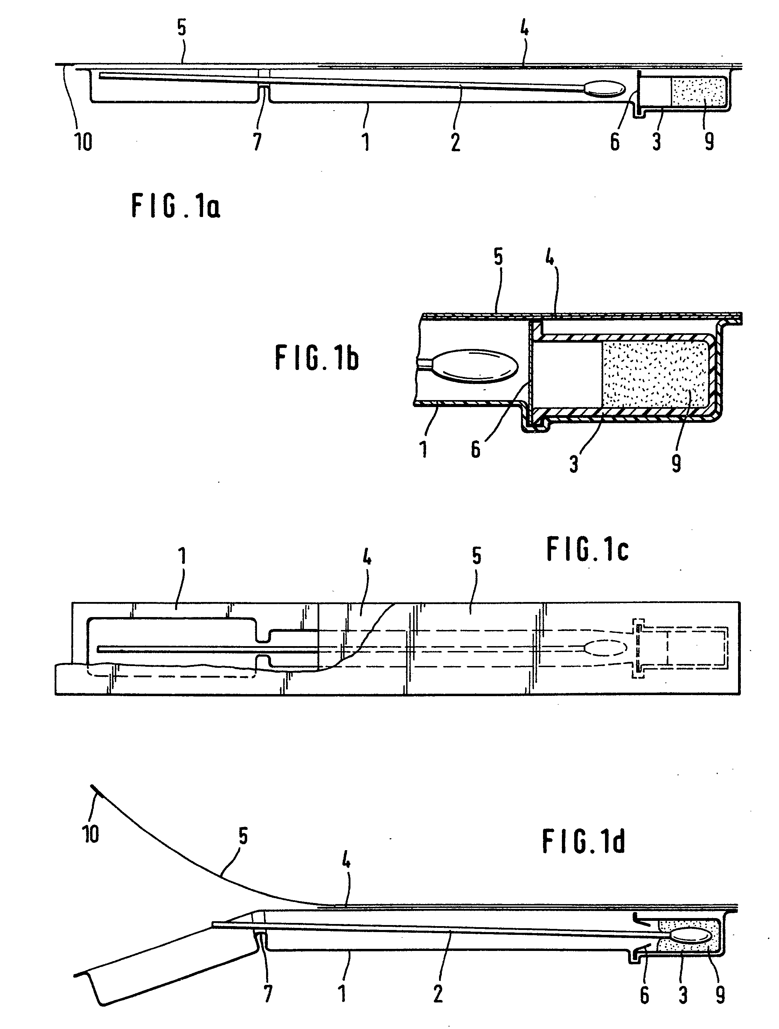

Die Erfindung wird anhand der Abbildungen 1-3 näher erläutert :

In Figur 1 a)-d) sind Seitenansichten und eine Aufsicht der erfindungsgemäßen Vorrichtung sowie die Einführung des Abstrichtupfers in den Kulturmediumbehälter dargestellt.

Der mit dem Kulturmedium (9) gefüllte Behälter (3) liegt zusammen mit dem Abstrichtupfer (2) lose, jedoch seitlich zentriert in dem Tiefziehteil (1) aus Kunststoffolie. Der Behälter (3) ist mit einer Aluminiumfolie (6) dicht verschlossen. Diese Folie ist je nach dem Werkstoff des Behälters aufgeschweißt oder aufgeklebt. Die Stärke der Aluminiumfolie (6) ist so gewählt, daß sie mit dem Abstrichtupfer (2) durchstoßen werden kann. Das Tiefziehteil (1) ist an der Oberseite im Bereich des Kulturmediumbehälters (3) und partiell im Bereich des Abstrichtupfers (2) mit einer aufgesiegelten Deckfolie (4) fest verschlossen. Über die gesamte Oberseite der Tiefziehpackung (1) ist eine einseitige Klebefolie (5) aufgebracht. Diese Klebefolie ragt einseitig als Angriffsstelle zum Aufziehen (10) ca. 8 mm über das Tiefziehteil (1) hinaus. In diesem Bereich ist die Klebeschicht abgedeckt. Das Tiefziehteil (1) ist an der Entnahmestelle (7) für den Tupfer eingeschnürt, um ein leichtes Wegbiegen des Tiefziehteilendstücks bei der Entnahme des Tupfers zu ermöglichen. Zur Entnahme des Tupfers wird das Klebeband bis zu einer markierten Stelle aufgezogen. Beim Anfassen des Stiels des Abstrichtupfers (2) wird das Endstück des Tiefziehteils nach unten weggebogen wie in Figur 1 d) dargestellt.

Nach dem Abstrich wird der Tupfer (2) wieder in das Tiefziehteil (1) eingeführt und der Wattebausch durch die Aluminiumfolie (6) in den Behälter mit Kulturmedium geschoben. Das Kulturmedium (9) sollte bei dieser Ausführungsart des Kulturmediumbehälters (3) eine relativ hohe Viskosität besitzen, damit der Abstrichtupfer beim Transport nicht aus dem Kulturmediumbehälter herausrutscht oder Kulturmedium aus dem Behälter (3) austritt. Nach Einführung des Tupfers in den Kulturmediumbehälter wird die Tiefziehpackung durch Andrücken der Klebefolie (5) wieder dicht verschlossen.

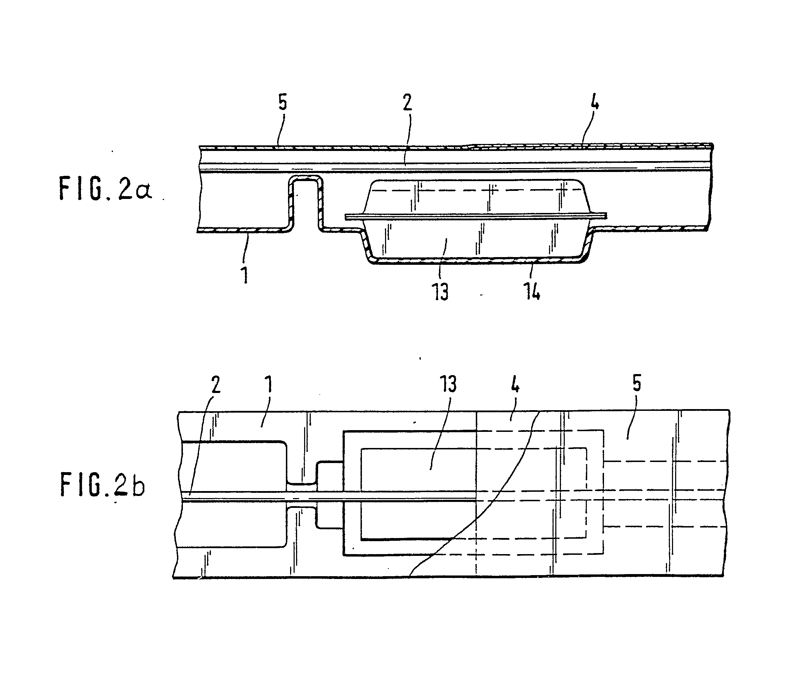

In Figur 2 a) und b) ist eine Seitenansicht und eine Aufsicht des mittleren Teils der erfindungsgemäßen Vorrichtung dargestellt, die zum anaeroben Transport von Mikroorganismen dient. Zu diesem Zweck enthält das Tiefziehteil (1) etwa in der Mitte eine muldenförmige Vertiefung (14) mit z. B. einem Beutel (13), der Reagenzien zur Erzeugung eines anaeroben Milieus enthält (z. B. Natriumcarbonat, Citronensäure und Eisenpulver auf einem Träger). Durch Zugabe geringer Mengen Wasser zum Reagenziengemisch reagiert vorhandener Sauerstoff mit dem Eisenpulver und gleichzeitig wird Kohlendioxid freigesetzt.

Die einseitig beschichtete Klebefolie (5) bietet z. B. ausreichend Platz für Produktinformationen sowie Daten zur Untersuchungsprobe, wie Patientenname, Untersuchungsmaterial, Einsender, Datum usw.

**Patentansprüche**

1. Sammel- und Transportvorrichtung für Mikroorganismen bestehend im wesentlichen aus einem einen Abstrichtupfer (2) und einen abgeschlossenen Kulturmediumbehälter (3) enthaltenden Tiefziehteil (1), das an der Oberseite teils mit einer aufgeschweißten Deckfolie (4) und mit einer abziehbaren und wieder dicht verschließbaren Klebefolie (5) verschlossen ist, dadurch gekennzeichnet, daß das Kulturmedium (9) in einem separaten abgeschlossenen, im Tiefziehteil (1) gelagerten Behälter (3) untergebracht ist und Behälter (3), Tiefziehteil (1), Deckfolie (4) und Klebefolie (5) aus Folien geringer Gas- und Wasserdampfdurchlässigkeit bestehen.

2. Sammel- und Transportvorrichtung nach Anspruch 1, dadurch gekennzeichnet, daß der Kulturmediumbehälter (3) aus einem mit einer aufgeschweißten oder aufgeklebten, mit dem Abstrichtupfer (2) durchstoßbaren Aluminiumfolie (6) verschlossenen Formteil aus Kunststoff besteht.

3. Sammel- und Transportvorrichtung nach den Ansprüchen 1 und 2, dadurch gekennzeichnet, daß das Tiefziehteil (1) an der Entnahmestelle (7) für den Abstrichtupfer (2) eingeschnürt ist, um das Wegbiegen des Tiefziehteilendstücks bei der Entnahme des Abstrichtupfers zu erleichtern.

4. Sammel- und Transportvorrichtung nach den Ansprüchen 1 bis 3, dadurch gekennzeichnet, daß das Tiefziehteil (1) im mittleren Bereich eine Vertiefung (14) zur Aufnahme eines Reagenziensystems (13) zur Erzeugung eines anaeroben Milieus aufweist.

## Claims

1. A device for collecting and transporting micro-organisms which essentially comprises a swab for taking smears (2) and a closed culture medium container (3) in a deep-drawn part (1), which is closed on its upper side partly by a covering film (4), which is welded on, and partly by an adhesive film (5) which can be pulled off and tightly closed again, characterised in that the culture medium (9) is accommodated in a separate, closed container (3), located in the deep-drawn part (1), and the container (3), deep-drawn part (1), covering film (4) and adhesive film (5) are composed of films of low permeability to gas and water vapour.

2. A device for collecting and transporting according to Claim 1, characterised in that the culture medium container (3) is composed of a moulded plastic part which is closed by an aluminum foil (6) which is welded on or glued on and can be penetrated by the swab for taking smears (2).

3. A device for collecting and transporting according to Claims 1 and 2, characterised in that the deep-drawn part (1) has a constriction at the point of removal (7) of the swab for taking smears (2) in order to facilitate bending away the end piece of the deep-drawn part on removal of the swab for taking smears.

4. A device for collecting and transporting according to Claims 1 to 3, characterised in that the deep-drawn part (1) has a depression (14) in the central zone to receive a reagent system (13) to produce an anaerobic environment.

## Revendications

1. Dispositif pour recueillir et transporter des micro-organismes, se composant essentiellement d'un boîtier embouti (1) contenant un tampon de prélèvement (2) et un récipient fermé pour milieu de culture (3), et qui est obturé sur le côté supérieur en partie au moyen d'une feuille de recouvrement soudée (4) et d'une feuille adhésive (5) pouvant être enlevée et à nouveau fermée de façon étanche, caractérisé en ce que le milieu de culture (9) est logé dans un récipient séparé et fermé (3) qui est disposé dans le boîtier (1) et en ce que le récipient (3), le boîtier embouti (1), la feuille de recouvrement (4) et la feuille adhésive (5) se composent de feuilles de faible perméabilité aux gaz et à la vapeur d'eau.

2. Dispositif pour recueillir et transporter selon la revendication 1, caractérisé en ce que le récipient pour milieu de culture (3) se compose d'une partie formée en matière plastique, qui est fermée au moyen d'une feuille d'aluminium (6) fixée par soudage ou par collage et pouvant être transpercée par le tampon de prélèvement (2).

3. Dispositif pour recueillir et transporter selon les revendications 1 et 2, caractérisé en ce que le boîtier embouti (1) est pourvu d'un étranglement dans la zone d'enlèvement (7) du tampon de prélèvement (2), afin de faciliter la flexion de la partie extrême du boîtier embouti lors de l'enlèvement du tampon de prélèvement.

4. Dispositif pour recueillir et transporter selon les revendications 1 à 3, caractérisé en ce que le boîtier embouti (1) comporte dans une zone centrale un évidement (14) pour recevoir un ensemble de réactifs (13) servant à produire un milieu anaérobie.

FIG.1a

FIG.1b

FIG.1c

FIG.1d

FIG.2a

FIG.2b